# EUROPEAN PATENT SPECIFICATION

(11) **EP 4 243 708 B1**
(45) Date of publication and mention of the grant of the patent: **25.02.2026**
(21) Application number: 21893087.3
(22) Date of filing: 12.11.2021
(51) Int. Cl.: A61B 17/22, A61B 17/3207, A61M 25/00, A61B 90/00, A61M 25/01, A61B 17/00

(54) **DISTAL ASPIRATION CATHETER**
DISTALER ASPIRATIONSKATHETER
CATHÉTER D'ASPIRATION DISTAL

(30) Priority: 13.11.2020 US 202063113757 P
(43) Date of publication of application: 20.09.2023
(73) Proprietor: Microvention, Inc., Aliso Viejo, California 92656 (US)
(72) Inventor: DHOLAKIA, Ronak, Aliso Viejo, California 92656 (US); RANGWALA, Hussain, Aliso Viejo, California 92656 (US)
(74) Representative: Bosch Jehle Patentanwaltsgesellschaft mbH
(86) International application number: PCT/US2021/072385
(87) International publication number: WO 2022/104374

(56) References cited:
- EP-A1- 2 548 601
- EP-A1- 3 708 199
- US-A- 5 938 645
- US-A1- 2007 250 096
- US-A1- 2013 060 206
- US-A1- 2015 190 668
- US-A1- 2018 280 045
- US-A1- 2018 338 770
- US-B2- 10 188 409
- US-B2- 10 292 721
- US-B2- 7 942 852

## Description

### RELATED APPLICATIONS

This application claims benefit of and priority to U.S. Provisional Applications Serial No. 63/113,757 filed August 21, 2020, entitled *Distal Aspiration Catheter And Methods* and Serial No. 63/086,200 filed October 1, 2020, entitled *Aspiration Thrombectomy Devices And Methods.*

### BACKGROUND OF THE INVENTION

Many diseases result from the presence of undesirable material, most notably clots or thrombus, in blood vessels and heart chambers. Clots within vessels can be formed from blood cells, collagen, cholesterol, plaque, fat, calcified plaque, bubbles, arterial tissue, aggregates of proteins (e.g., fibrin), and/or other miscellaneous fragments or combinations thereof. Clots can lodge, for example, in narrow regions of blood vessels that feed the major organs, and therefore, cause loss of oxygen-rich blood flow to surrounding tissues resulting in localized cell death or micro-infarcts. Cerebral micro-infarcts are ischemic strokes that typically lead to confusion, disturbance of speech, paralysis, visual disturbances, balance disturbances and even death. In the heart, clots can cause myocardial infarcts, i.e., heart attacks. If the clots are left untreated, they can be potentially life threatening, and therefore, the presence of clots in blood vessels need immediate medical intervention.

Clots can typically be treated or eliminated by utilizing biologic intervention, surgical intervention, or a combination of the two. Biologic treatments involve the delivery of agents directly to the clots by a catheter to either dissolve or at least stabilize it until the body can eliminate it. However, the disadvantage of treating clots with biologic agents is that they expose much of the body to the agents, potentially leading to life-threatening bleeding complications.

Alternately or additionally, mechanical means can be used to remove the clots from a patient's vessel. Mechanical treatments typically involve aspiration, maceration, and compression of the clots within the vessel and finally removing it either by invasive surgery or by non-invasive means, such as with an aspiration catheter connected to an aspiration source (e.g., pump or syringe). The distinct advantage of invasive or non-invasive mechanical treatment is that it directly attacks the clots and eliminates the vascular obstruction without affecting non-diseased areas of the body, unlike biologic agents.

The mechanical, non-invasive clot removal treatments generally relate to thrombectomy procedures, more particularly, peripheral thrombectomy procedures and venous thrombectomy procedures, such as deep venous thrombosis (DVT) treatment or intracranial distal aspiration.

Acute ischemic stroke treatment using vascular aspiration embolectomy involves aspiration techniques to draw clots into a catheter via suction and has been proven to be as effective as mechanical thrombectomy using stent retrievers which deploy self-expanding stent-like capture devices to capture and remove the thrombus. Aspiration embolectomy has gained favor with a major section of neuro-interventional physicians due to the ease of the procedure and its cost effectiveness.

In aspiration embolectomy, a distal aspiration catheter (DAC) is used to remove the clot or emboli from the vessel. At the first step of the procedure, a distal end of the catheter is positioned adjacent to the clot and then a vacuum force is applied through a lumen in the catheter using a pump or large volume syringe. The suction force used by the syringe or pump partially ingests or pulls in the emboli into the distal portion of the DAC. In such instances, when the emboli are partially ingested, repeated passes of the device may be needed to recanalize the vessel.

Successful clinical outcome of the patients undergoing aspiration embolectomy, however, may in part depend on the "first -pass effect". The first-pass effect is defined as the complete or near-complete recanalization of the affected vessel after one pass of an embolectomy device by complete removal of the emboli in the first attempt.

Inside the vessels, however, blood clots can undergo a process called organization where the soft gel-like red/purple clot is transformed into a firmer, whitish clot by the cross-linking of proteins such as fibrin. Clots may also form firm masses with the forward pressure of blood flow over time. Firm clots can form a large mass of well embedded, mature clots over time which are less flexible and can be difficult to completely ingest into the traditional DAC when static suction is applied with the attached pump or aspiration syringe kit. This partial ingestion of thrombus/emboli using a static suction of a traditional DAC, therefore, reduces the chances of a first pass effect and requires additional passes of the traditional DAC for complete removal of thrombus/emboli.

Additionally, there is also the possibility of fragmentation of a well embedded, mature thrombus/emboli during removal which may allow fragments to travel to other areas in the blood vessels. The above risk factors for removing a large mass of well embedded, mature clots by using a traditional DAC negatively impacts the clinical outcome of a patient by reducing the chance of "first pass effect".

Furthermore, the use of a stent retriever in combination with a traditional DAC, known as the Solumbra technique, has become a popular approach to mechanical thrombectomy for acute ischemic stroke. However, the Solumbra technique has not been shown to improve the first pass effect over aspiration embolectomy alone.

EP 3708199 A1 discloses a tissue capturing cannula having a distal chamber and a side opening.

US 7942852 B2 discloses a clot aspiration catheter having a side port and another lumen distally from the side port.

Hence, there is a need for an improved DAC which can at least overcome the problems discussed above for a traditional DAC which uses static aspiration, particularly for removing well embedded, mature clots and improving the clinical outcome in patients by complete recanalization of the vessel after one pass of the DAC device.

### SUMMARY OF THE INVENTION

The present invention is directed to an aspiration catheter to fatigue a clot so that it can be completely removed from a blood vessel. These catheter devices may be particularly helpful for firm and/or well-embedded clots that might otherwise present difficulties during a removal process. In some instances, these embodiments can lead to recanalization of the blood vessel after one pass of the catheter.

The invention is defined by claim 1. Further embodiments of the invention are defined by the dependent claims.

In one embodiment, the aspiration catheter comprises a catheter body having a catheter tip that can be used to fatigue a clot and a clot-holding cavity, chamber, or lumen into which a clot can be position after entering the catheter. In one example, the catheter tip is located at a distal end of the catheter and the clot holding lumen is located at least partially within the catheter tip. The clot-holding lumen is connected to or in communication with a main aspiration lumen that extends between a proximal end and a distal end of the catheter, and at least one distal opening is positioned proximal of the distal end of the tip to allow a clot to be pulled into the catheter and positioned within the clot-holding lumen.

The catheter tip can be relatively smooth or can have a shape configured to assist in penetrating or softening the clot, such as a helical spiral shape. The one or more distal openings can be a single opening that is radially offset from the center of the catheter tip or can be a plurality of openings disposed radially around a distal portion of the catheter.

Generally, the catheter tip can be first advanced into or adjacent to the clot and aspiration can be applied to direct the clot into the distal opening of the catheter and then into the clot-holding lumen. In one example, static aspiration is applied through the main aspiration lumen of the catheter to the distal opening, causing the clot to at least partially move into the distal opening. Cyclic aspiration can be applied to assist in the fatiguing or weakening the clot and further moving the clot into the main aspiration lumen of the catheter. Since the cyclic aspiration tends to cause proximal force when aspiration is activated and a small return distal force when deactivated, the cyclic aspiration may tend to migrate the clot at least partially into the clot-holding lumen within the tip of the catheter. Once the clot has been partially or fully ingested by the catheter, the catheter and clot can both be removed from the patient.

### BRIEF DESCRIPTION OF THE DRAWINGS

These and other aspects, features and advantages of which embodiments of the invention are capable of will be apparent and elucidated from the following description of embodiments of the present invention, reference being made to the accompanying drawings, in which:
Fig. 1 illustrates a perspective view of an aspiration embolectomy as a treatment for acute ischemic stroke using a traditional DAC in which the clot is partially ingested after application of a suction force.
Fig. 2 illustrates a perspective view of a DAC in one embodiment of the present invention.
Fig. 3 illustrates a side elevation of a distal portion of an embodiment of a DAC of the invention.
Fig. 4 illustrates an end view of a distal portion of an embodiment of a DAC of the present invention.
Fig. 5A illustrates a step of a method of using an embodiment of a DAC to perform an embolectomy.
Fig. 5B illustrates a step of a method of using an embodiment of a DAC to perform an embolectomy.
Fig. 5C illustrates a step of a method of using an embodiment of a DAC to perform an embolectomy.
Fig. 6 illustrates a perspective view of a DAC in one embodiment of the present invention.
Fig. 7 illustrates a perspective view of a distal portion of an embodiment of a DAC of the present invention.
Fig. 8 illustrates a perspective view of a DAC in one embodiment.
Fig. 9 illustrates a perspective view of a DAC in one embodiment.
Fig. 10 illustrates a step of a method of using an embodiment of a DAC to perform an embolectomy.
Fig. 11 illustrates a step of a method of using an embodiment of a DAC to perform an embolectomy.
Fig. 12 illustrates a perspective view of a DAC in one embodiment of the present invention.
Fig. 13 illustrates a perspective view of a DAC in one embodiment.

### DETAILED DESCRIPTION

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements. While different embodiments are described, features of each embodiment can be used interchangeably with other described embodiments. In other words, any of the features of each of the embodiments can be mixed and matched with each other, and embodiments should not necessarily be rigidly interpreted to only include the features shown or described.

The terms clot, thrombus, and embolus are used interchangeably within this specification. Generally, these terms mean a mass or connected group of blood cells, collagen, cholesterol, plaque, fat, calcified plaque, bubbles, arterial tissue, aggregates of proteins (e.g., fibrin), and/or other miscellaneous fragments or combinations thereof.

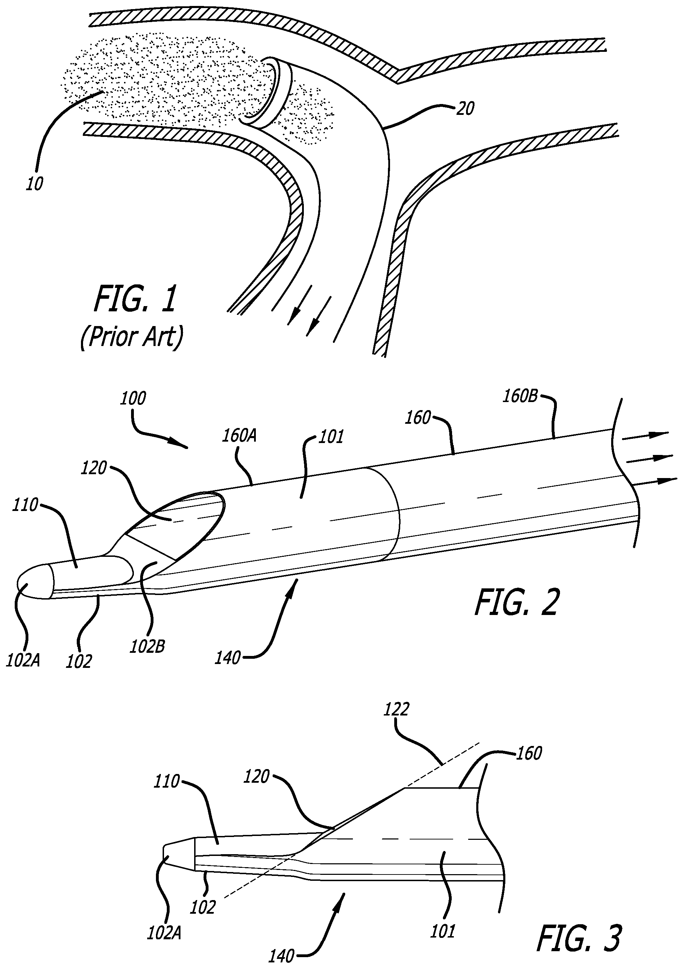

As previously discussed, existing aspiration catheter techniques using only a static suction force may not be effective in removing a large amount of firmly embedded clot from inside a blood vessel, particularly after one pass of an aspiration catheter because the clot is unable to be pulled completely inside the aspiration catheter. For example, Fig. 1 illustrates a traditional distal access catheter (DAC) 20 that is used to remove a large clot 10 from inside the vessel. The DAC 20 might typically have an inner diameter profile ranging from 1.3 (4F) to 4 (12F) mm. In the present example, the clot 10 is lodged in a middle cerebral artery causing large vessel occlusion and acute ischemic stroke. During the removal procedure, the distal end of the DAC 20 is positioned adjacent to the clot 10 and the clot is partially ingested or suctioned inside a lumen of the distal portion of the DAC 20 after a vacuum force is applied at a proximal end of the lumen with an attached pump or aspiration syringe.

If the clot 10 is firmly embedded and/or larger than the diameter of the DAC 20, applying vacuum force alone may not be effective in complete removal of the clot 10 and recanalization of the vessel. In other words, complete removal of the clot 10 after a first pass of the device 20 may not be possible. While it may be possible to use a larger diameter DAC and/or apply a stronger vacuum force, a larger profile DAC can be more challenging to navigate to achieve distal intracranial circulation and may be detrimental to the vessel lumen, causing dissection or vasospasm.

One aspect of the present invention seeks to address the deficiencies of the existing devices for removing a clot after one pass of a DAC during a procedure. Some of the embodiments described herein are directed to a DAC (also referred to more generally as a catheter) that can include certain structural features used in connection with cyclic aspiration to fatigue a clot, move the clot inside the catheter, and/or safely capture the clot within the DAC for its safe removal from the patient.

The term "clot fatigue" is generally defined as a process to apply mechanical stress to a clot to alter or reduce stiffness, size, and/or cohesion to cause the clot to partially or completely break down. Performing procedures to fatigue a clot can be particularly helpful for aspirating a clot into a catheter and removal from a patient.

The DAC of the present invention can be used to remove clots and treat vessel occlusion for any kind of blood vessels. For example, the DAC of the present invention can be used to treat vessel occlusion caused by acute ischemic stroke, peripheral artery thrombosis, pulmonary embolism (e.g., pulmonary arteries), deep vein thrombosis, systemic venous circulation (e.g., jugular vein, sigmoid sinus, transverse sinus, superior and inferior sagittal sinus, vena cava, pelvic veins, femoral veins, or subclavian veins), or arterial circulation (e.g., aorta or its large and medium branches).

The DAC comprises a distal tip that extends distally of a distal opening of an aspiration lumen of the DAC. The distal tip is configured to help physically direct a clot into one or more distal openings of the aspiration lumen and/or to induce clot fatigue through physical contact. For example, the distal tip can have a relatively smooth conical shape, a flattened conical shape (e.g., a generally oval cross section), a conical shape with a depression extending axially toward the distal opening of the aspiration lumen, a triangular shape, a relatively rectangular shape, a helical shape, a conical helical shape, a conical shape with a plurality of axial channels extending axially toward the distal opening(s) of the aspiration lumen, a cylindrical shape, or any combination or similar variation thereof.

The DAC can include one or more distal openings of its aspiration lumen. The one or more distal openings can be located proximally of the distal tip (e.g., between about 5 mm and 25 mm from the very distal end of the distal tip). In the example of a single distal opening, the opening can be angled distally, laterally, or an angle that is both distally and laterally (e.g., about 45 degrees relative to an axis of the DAC). In one example, the single distal opening may be positioned off axis or in a non-radially symmetric location. In other words, the distal opening may be positioned closer to one side of the DAC, which may optionally allow the distal tip to be positioned closer to an opposite side of the DAC.

In another example, a plurality of distal openings can be included into the aspiration lumen of the DAC. For example, 2, 3, 4, 5, 6, 7, 8, or more openings can be included. These openings can all be positioned at different circumferential positions but at the same longitudinal positions, or at different circumferential positions but at two or more different longitudinal positions (i.e., some openings are more proximal of other openings).

The one or more openings may have a variety of different shapes, such as oval, square, or rectangular. Additionally, the openings can be surrounded by uniform portions of the outer DAC wall or can be located within a depression or channel in the outer DAC wall that is configured to help direct portions of the clot into one or more of the distal openings.

In any of the embodiments of this specification, the DAC may include a valley or longitudinal depression extending along at least a portion of the length of the distal tip. This valley or longitudinal depression may also include one or more openings that open into the interior cavity or the aspiration lumen. These openings may help draw a clot against the tip and then into the distal opening. In the case of multiple openings, these openings may be positioned longitudinally along the length of the longitudinal depression. These openings may also all have the same diameter or have different diameters, such as increasing diameters in the proximal direction to help draw a clot to the distal opening. Alternately, a valley or longitudinal depression may not be included and the openings may instead be located along a surface of the distal tip, aligned with or at least near the distal opening into the aspiration lumen.

The aspiration lumen generally comprises a passage between the distal end of the DAC, including the distal openings, and a proximal end of the DAC (e.g., a port on a catheter hub of the DAC). An aspiration source, such as a syringe or motorized aspiration device, can be connected to or put into communication with the proximal end of the aspiration lumen, allowing selective aspiration to be applied to the aspiration lumen.

The aspiration source, particularly a motorized aspiration device, can be configured to apply cyclic aspiration to the aspiration lumen during a procedure. Cyclic aspiration generally can include increasing and decreasing an aspiration or vacuum level in relatively quick succession. For example, the aspiration level can be activated and then deactivated, or aspiration can instead be varied between weaker and stronger levels. In another example, the cycling of the aspiration can occur within intervals of .5 seconds to 3 seconds (e.g., intervals of .5, 1, 1.5, 2, 2.5, or 3, as well as combinations thereof).

With further regard to cyclic aspiration, the DAC may include one or more features that, when used with cyclic aspiration help contain or capture the clot within the DAC. For example, the distal portion of the DAC may be partially or completely hollow and include surfaces that help bring the clot into the aspiration lumen upon increased aspiration and release the clot into the interior lumen or cavity of the distal portion upon decrease or cessation of the aspiration. Hence, the clot may be "trapped" in an interior cavity of the distal portion of the DAC and is therefore less likely to move out of or fall out when the DAC is retracted from the patient.

One specific embodiment of a DAC in accordance with the present claimed invention, is seen in Figs. 2-5C and includes a distal portion 140 of the DAC 100 comprising a distal tip 102, as well as a distal opening 120 of an aspiration lumen 101 of the DAC 100. As discussed further below, the distal tip 102 and the distal opening 120 are positioned on opposite sides of a longitudinal axis of the DAC 100 so that the tip 102 can be used to optionally fatigue the clot, as well as help guide it into the distal opening 120. Additionally, the distal tip 102 includes a lumen or interior cavity 110 within the DAC 100 that is in communication with the aspiration lumen 101 and into which a clot can be partially or fully pulled into, particularly when using cyclic aspiration. This interior cavity 110 may also be referred to as an interior clot-holding cavity.

The distal tip 102 generally forms a distalmost structure at the end of the distal portion 140 of the DAC 100. In the present embodiment, the distal tip 102 is positioned towards one side of the DAC 100, offset from a longitudinal axis of the DAC 100. This offset position may allow the distal tip 102 to smoothly navigate around or to the side of a clot so that the clot can be easily positioned against the distal opening 120. The rounded distal end 102A and angled transition area 102B adjacent to the distal opening 120 may further help position the clot against the distal opening 120, prior to or during activation of aspiration through the aspiration lumen 101.

However, depending on the shape of the distal tip 102, the distal tip 102 may alternately be positioned generally radially symmetrically around or partially around the longitudinal axis of the DAC 100 (i.e., at a center of the device).

The distal tip 102 of the present embodiment has a generally conical or cylindrical shape that terminates with a closed, rounded end 102A. However, it may alternately have a flattened conical shape (e.g., a generally oval cross section), a conical shape with a depression or valley into the outer surface and extending axially toward the distal opening 120 of the aspiration lumen 101, a triangular shape, a relatively rectangular shape, a helical shape, a conical helical shape, a cylindrical shape, or any combination or similar variation thereof. The distal tip may have a length within a range of about 1 mm to 5 mm.

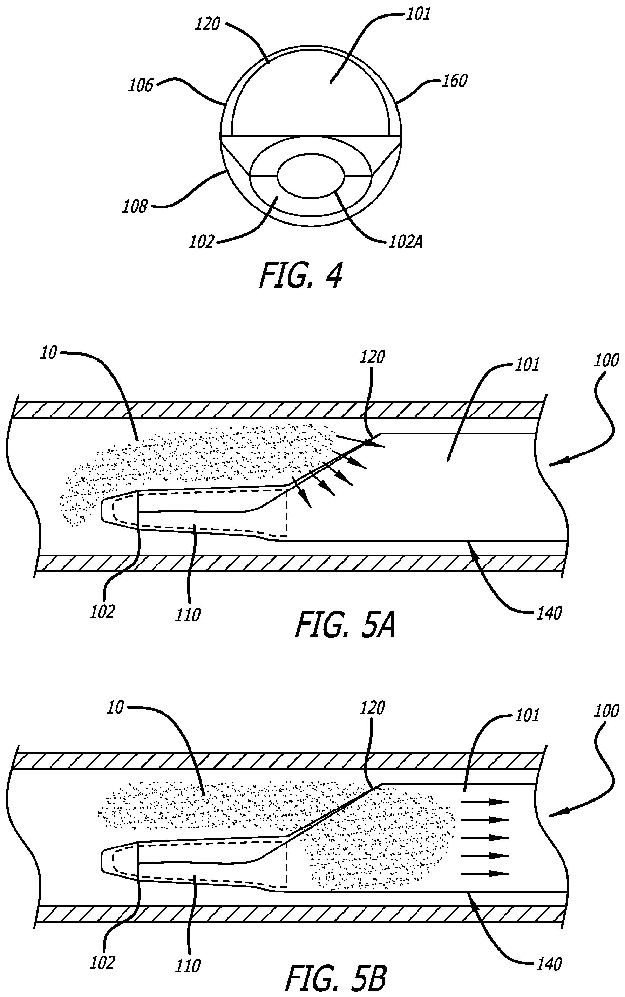

In some embodiments, the distal portion 140 can include an interior or inner lumen, chamber, or cavity 110 that is located at least partially distal of the distal opening 120 and is in communication with the aspiration lumen 101, which allows the interior cavity 110 to be used to at least partially capture a clot, particularly when cyclical aspiration is used, as discussed later in this specification. In the present example embodiment, the interior cavity 110 is located at least partially within a hollow distal tip 102 as shown in dotted lines in Figs. 5A-5C and forms a continuous passage with the aspiration lumen 101. The interior cavity 110 can extend the entire length of the distal tip 102 or only along part of its length. The interior cavity is shown as having a relatively uniform, cylindrical or conical shape, but other shapes, such as an oval cross-sectional shape are possible. Additionally, the interior surface of the interior cavity 110 may have bumps, rings, waves, hooks, pins, or similar features to help engage the clot once in position.

The distal opening 120 of the aspiration lumen 101 can be positioned near a proximal end of the distal tip 102. In the present embodiment, the distal opening 120 is positioned towards one side of the DAC 100, offset from a longitudinal axis of the DAC 100 and opposite the side of the distal tip 102. However, depending on the position and shape of the distal tip 102, the distal opening 120 may alternately be positioned generally radially symmetrically around the longitudinal axis of the DAC 100 (i.e., at a center of the device, partially or fully around the distal tip).

The distal opening 120 can have a generally half circle shape to help maximize the size of the distal opening 120, however different shapes of smaller or larger sizes are possible, such as circular or oval.

The distal opening 120 also may form its opening at various angles relative to the longitudinal axis of the DAC 100. In the present embodiment, the distal opening 120 is bias cut or forms a plane 122 at about 45 degrees relative to the longitudinal axis of the DAC 100 (seen best in Fig. 3). The transition area 102B shown in Fig. 2 may form a surface having a similar angle to that of the distal opening 120, or a larger or smaller angle. Other angles of the distal opening 120 are also possible, such as 90 degrees (i.e., perpendicular), 315 degrees (i.e., the opposite angle of Fig. 2), 15 degrees, 25 degrees, 75 degrees, or any angle within 10 percent of those values.

The 45-degree distal opening 120 of Fig. 3 or similar angles that proximally increase in height may help slide against a clot to better position the clot against the distal opening 120. Angles such as 315 degrees that are proximally angled inward towards the cross-sectional center of the DAC 100 may extend at least partially parallel and opposite of the distal tip 102 and may therefore engage the clot on opposite sides.

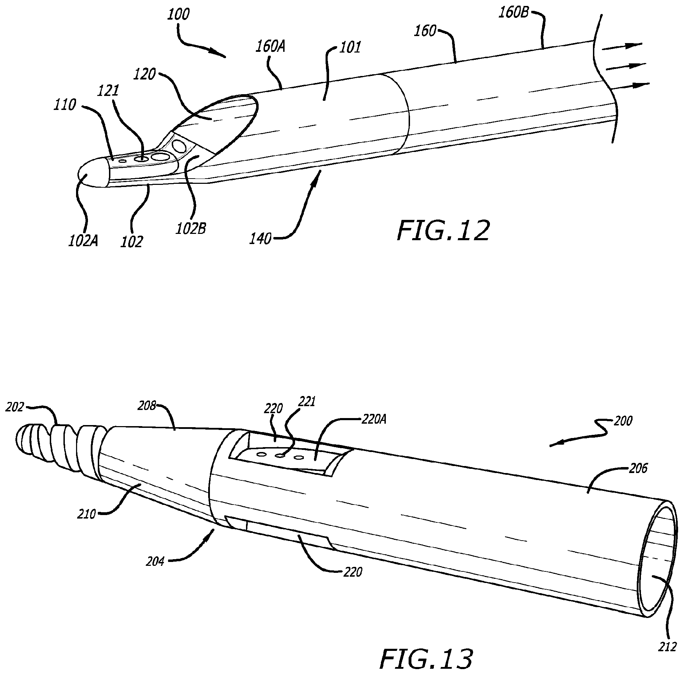

As seen in Fig. 12, in some embodiments, the DAC 100 may include a valley or longitudinal depression extending along at least a portion of the length of the distal tip 102. This valley or longitudinal depression may also include one or more openings 121 that open into the interior cavity 110 or the aspiration lumen 101. These openings 121 may help draw a clot against the tip 102 and then into the distal opening 120. In the case of multiple openings 121, these openings 121 may be positioned longitudinally along the length of the longitudinal depression. These openings 121 may also all have the same diameter or have different diameters, such as increasing diameters in the proximal direction to help draw a clot to the distal opening 120. Alternately, a valley or longitudinal depression may not be included and the openings 121 may instead be located along a surface of the distal tip 102, aligned with or at least near the distal opening 120 into the aspiration lumen 101.

The DAC 100 can be created according to known catheter manufacturing techniques such that it at least has an outer tubular wall 160 that forms the aspiration lumen 101 that extends between the distal opening 120 and a proximal end of the DAC 100 (e.g., a port on a catheter hub of the DAC 100, not shown). It may be desirable for the DAC 100 to increase in flexibility towards its distal end relative to its proximal end. This can be achieved by composing different portions of the catheter wall 160 with different materials. For example, a proximal wall portion 160B may include stiffer materials relative to a distal wall portion 160, and distal tip 102 and/or the rounded distal end 102A may be composed of even more flexible material.

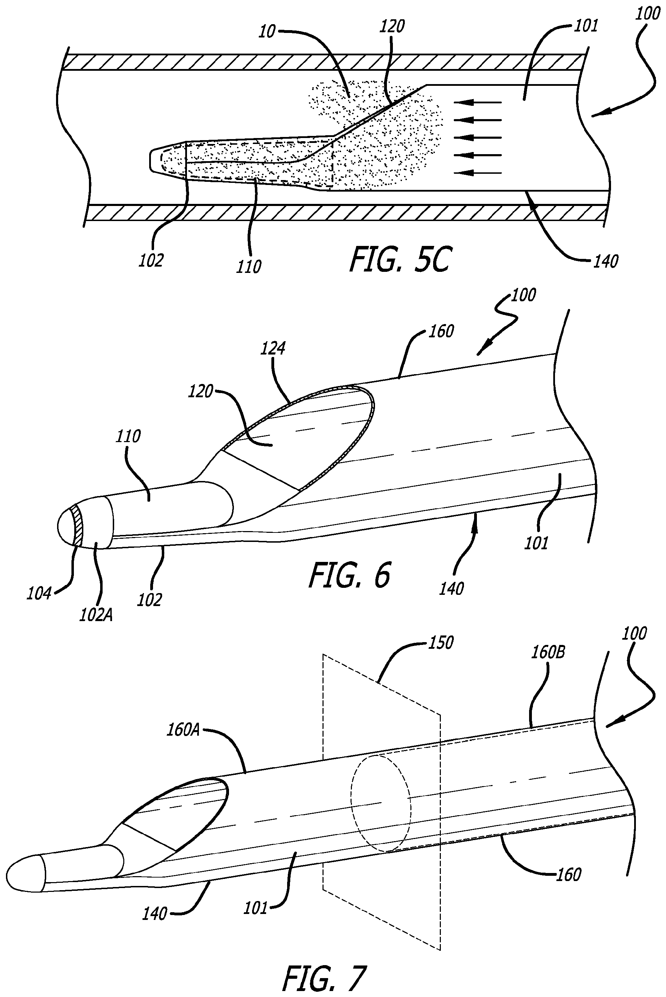

One way to achieve the difference in stiffness is to fuse a tube of a first material with a first hardness to create the proximal wall portion 160B with another tube of a second material to create the distal wall portion 160A, as seen in Fig. 7. The proximal wall portion 160B can be fused with the distal wall portion 160A at the plane 150 in such a way that the diameter of the proximal portion 160 and the diameter of the distal portion 140 are similar or identical. The distal portion 160A of the DAC 100 can be made of any material which is kink resistant and provides softness during navigation without compromising the durability of the DAC 100, such as PeBax or other thermoplastics. The proximal portion 160B of the DAC 100 may comprise polymers which are braided and reinforced to increase the stiffness of the proximal portion 160, such as PTFE, which may provide increased stiffness to better attach the proximal wall portion 160B with a handle (not shown in the drawings).

Optionally, radiopaque markers can be included on any of the DACs of this specification. These markers can be located at or near the very distal end of the DAC, around or near the distal opening, and one or more locations proximal of the distal opening. Returning to the embodiment of the DAC 100, Figure 6 illustrates a first radiopaque marker 104 that is positioned on or within the distal end portion 102A of the distal tip 102. Alternately or additionally, the marker 104 can be located at other locations along the distal tip 102.

The distal opening 120 of the DAC 100 can include a radiopaque marker 124 positioned at or along the edge of the distal opening 120, as seen in Fig. 6. This may allow a physician to see the angle of the opening and its relative distance from radiopaque marker 104. Alternately, the marker 124 can be located on or embedded in the distal wall portion 160A, near distal or proximal end of the distal opening 120. The radiopaque marker bands 104 and 124 may be composed of radiopaque materials, platinum, tantalum, or similar metals.

The DACs of this specification can be used with static aspiration (i.e., a constant aspiration vacuum force), cyclic aspiration force (i.e., aspiration force that cycles between higher and lower/no aspiration force), or combinations of both to completely remove the clot 10 and recanalization of the blood vessel in which the clot is lodged.

In the case of cyclic aspiration, a phenomenon commonly referred to as a "water-hammer effect" can sometimes be achieved. Typically, the water-hammer effect refers to a pressure surge or high-pressure shockwave in liquid conduits that propagates through a piping system when a fluid in motion is forced to change direction or stop abruptly. In the context of the present invention, this pressure surge or shockwave may cause the fluid within the aspiration lumen to move in a direction opposite of the aspiration for a very short time. In other words, during aspiration, fluid will move proximally toward the aspiration source within the aspiration lumen and when aspiration is abruptly stopped or reduced, a pressure shockwave may push the clot (and potentially some fluid) back in a distal direction.

The outcome of this water-hammer effect can best be seen in the DAC 100 embodiment of Figures 5A-5C. In Fig. 5A, aspiration is activated to move the clot 10 proximally until it at least partially moves into the distal opening 120. When aspiration is turned off or decreased abruptly, a pressure shockwave is created according to the arrows in Fig. 5C. This pushes the clot into the interior cavity 110 of the distal tip 102. This aspiration cycle to achieve the water hammer effect may only be necessary once, or may be performed many times (e.g., 2, 3, 4, 5, 6, 7, 8, or more times) to position the clot 10 in the desired position in the interior cavity 110. This position of the clot 10 within the interior cavity 110 may more firmly capture the clot 10 and therefor may better prevent the clot 10 from slipping out of the DAC 100 when being withdrawn from a patient.

It should be noted that an alternative to this water hammer pressure wave might be achieved, at least for the purposes of moving the clot into the interior cavity 110, by applying a period of initial, proximal aspiration, followed by a short period of distal pressure in the distal direction (i.e., reverse flow in the aspiration lumen 101).

For completeness, a method of using the DAC 100 of Figs. 2-7 is described further below. However, it should be understood that this method can be performed with any of the embodiments and/or variations described in this specification.

A guidewire and/or delivery sheath (not shown in the drawings with DAC 100) may be initially deployed in the patient such that the guidewire or sheath allows the distal tip 102 of the DAC 100 to be positioned near a clot 10. For example, the DAC 100 may be inserted into the targeted blood vessel over the guidewire (e.g., through the aspiration lumen 101 or a separate guidewire lumen) and advanced towards the clot 10 or the DAC 100 can be advanced within a placed sheath having its distal end placed near the clot 10. The radiopaque marker bands 104, 124 of the DAC 100 can be used to confirm the proper positioning of the DAC 100 with respect to the clot 10.

The DAC 100 is further distally advanced so that the distal tip 102 is positioned to the side of the clot 10, as seen in Fig. 5A. Alternately or additionally, the distal tip 102 may be positioned into or through the clot 10, depending on the shape of the distal tip 102. Optionally, the distal tip 102 may be moved (e.g., proximally, distally, or laterally) to physically move the clot to induce clot fatigue.

Once the clot 10 is positioned near or against the distal opening 120, the aspiration source connected to the proximal end of the aspiration lumen 101 is activated such that the clot 10 is ingested at least partially into the aspiration lumen 101 of the DAC 100 (as shown in Fig. 5B).

This may be achieved by static aspiration or through cyclic aspiration. Applying cyclic aspiration at this time may help further fatigue the clot 10, especially if it is sized and structured such that it is resistant to passing into the distal opening 120. Particularly, the water hammer pressure waves from this cyclic aspiration may help create a dynamic normal force ranging from 0-1 N on the clot such that clot fatigue is induced. Generally, a force above 0.5 N should be appropriate for most situations. It is important to note that these pressure values are not static force but are instead applied momentarily and in the form of a dynamic/sharp ramp characterized by the water hammer pressure waves. Hence, it may be desirable to perform an initial period of cyclic aspiration to create pressure waves to fatigue the clot 10, then apply a period of static aspiration to suction at least part of the clot 10 into the aspiration lumen 101.

Once the clot 10 is at least partially ingested into the aspiration lumen 101 (Fig. 5B), an additional period of cyclic aspiration may be performed. The water hammer pressure waves created with this cyclic aspiration may push part or all of the clot 10 distally forward into the interior cavity 110 of the distal tip 102, as seen in Fig. 5C. This positioning of the clot 10 within the interior cavity 110 helps hold or secure the clot in place, which may be particularly helpful as the DAC 100 is proximally withdrawn from the patient's vessel. In some circumstances, this withdrawal processes can create forces on the clot 10 if a portion of the clot remains outside of the DAC 100 or if distal suction is created by the proximal movement of the DAC 100.

After the DAC 100 is withdrawn, a contrast agent may be injected to determine if the recanalization of the blood vessel was successful.

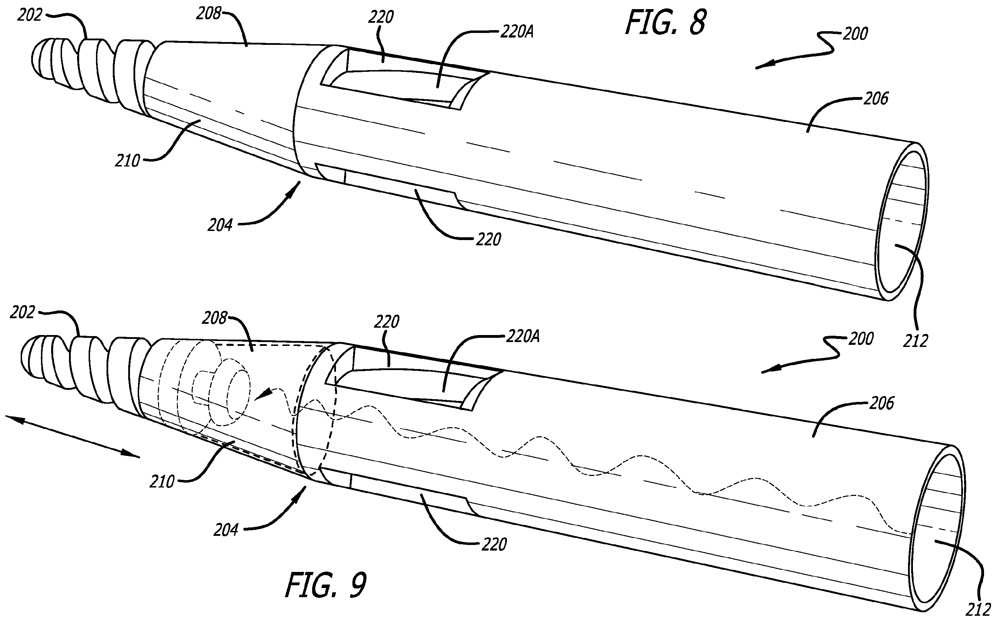

As previously discussed, the tip of the DAC can have a variety of different shapes and a plurality of distal openings into the aspiration lumen of the DAC. Figs. 8 and 9 illustrate such an embodiment of a DAC 200 that is generally similar in structure and function to the previously described DAC 100, but a distal portion 204 of the DAC 200 can include a distal tip 202 that includes a spiral shape. Additionally, a plurality of distal openings 220 into an aspiration lumen 212 of the catheter body 206 can be included.

The distal tip 202 has a generally spiral or helical shape and is positioned at the distalmost end of DAC 200. Put another way, the distal tip 202 has a generally conical shape with a spiral groove or raised threads that helically spiral along the length of the distal tip 202. In one example, the distal tip can be between about 2 mm and about 10 mm. This distal tip 202 is preferably composed of a somewhat soft material that reduces or prevents trauma to a patient's vessel.

The distal tip 202 is connected to an intermediate conical portion 208 that creates a relatively uniform connection interface between the smaller diameter distal tip 202 and the larger diameter catheter body 206. Similar to the previously described DAC 100, the intermediate conical portion 208 may form an internal or interior cavity 210 within it and distal to the distal openings 220 to allow clot 10 to be positioned within (this interior cavity 210 is denoted by dotted lines in the figures). The conical portion 208 can be between about 3 mm and about 15 mm in length.

The interior cavity 210 can extend the entire length of the distal tip 202 or only along part of its length. The interior cavity is shown as having a relatively uniform, cylindrical or conical shape, but other shapes, such as an oval cross-sectional shape are possible. Additionally, the interior surface of the interior cavity 210 may have bumps, rings, waves, hooks, pins, or similar features to help engage the clot once in position.

In the present embodiment, the distal tip 202 is fixed from rotation relative to the intermediate conical portion 208 and catheter body 206. In some cases, it may be helpful to at least partially rotate the entire DAC 200 to help engage and move the clot 10 towards the distal openings 220. However, the DAC 200 may also be configured such that the distal tip 202 rotates relative to the intermediate conical portion 208 and/or catheter body 206. In such an embodiment, a shaft may be connected to the distal tip 202 and may extend to a proximal end of the catheter body 206 where the shaft can be rotated by hand or can be connected to a motorized rotational mechanism.

The plurality of distal openings 220 open to the aspiration lumen 212 of the catheter body 206 and allow a clot 10 to be at least partially suctioned therethrough. In the present embodiment, three distal openings 220 included, though 2, 3, 4, 5, 6, 7, 8, or more distal openings 220 are also possible. The present distal openings 220 are all located at the same longitudinal positions at the distal portion 204 of the catheter body 206, however, it is also contemplated that one or more of these distal openings 220 may be positioned longitudinally distal or proximal to others.

The distal openings 220 of the present DAC 200 have a generally rectangular shape, however, other shapes are possible. For example, circular, oval, square, and similar shapes are possible. In order to ingest relatively large clots 10, it is desirable that each distal opening have a relatively large size. One way to achieve this with a plurality of distal openings 220 is to configure each distal opening 220 with a longer length along an axis of the DAC 200 relative to its width along a circumference of the DAC 200. Hence, more openings 220 can be included around the circumference of the DAC 200 while still being able to accept or ingest a relatively large clot.

In addition to the diametric shape, other shapes or design features can be included as part of the distal openings 220 that assist with moving the clot 10 in a desired manner into the aspiration lumen 212. For example, the present distal openings 220 form a recessed area or groove with a lower surface 220A. The lower surface 220A can be angled radially inwardly in a distal direction towards an opening into the aspiration lumen 212 at a distal end of the recessed area. In this respect, the distally slowed lower surface 220A may help direct a clot 10 into the aspiration lumen 212 in a distal direction such that when cyclic aspiration is applied to create a water hammer pressure wave, the clot 10 better moves into the interior cavity 210 of the intermediate conical portion 208. Alternately, the distal openings 220 may be a direct opening to the aspiration lumen 212.

As seen in Fig. 13, in some embodiments, the recessed area or groove with a lower surface 220A may also include one or more openings 221 that open into the interior cavity 210 or the aspiration lumen 212. These openings 221 may help draw a clot towards the lower surface 220A and then into the aspiration lumen 212. In the case of multiple openings 221, these openings 221 may be positioned longitudinally along the length of the lower surface 220A. These openings 121 may also all have the same diameter or have different diameters, such as increasing diameters in the proximal direction to help draw a clot into the aspiration lumen 212. Alternately, the openings 221 may be instead located along a surface of the conical portion 208, aligned with or at least near the distal opening 220 into the aspiration lumen 212.

The distal openings 220 are depicted on a cylindrical side of the catheter body 206. However, one or more may be located on a conical, distal surface, such as the intermediate conical portion 208.

As with previous embodiments, radiopaque markers can be included near or in the distal tip 202, as well as adjacent to the plurality of distal openings 220.

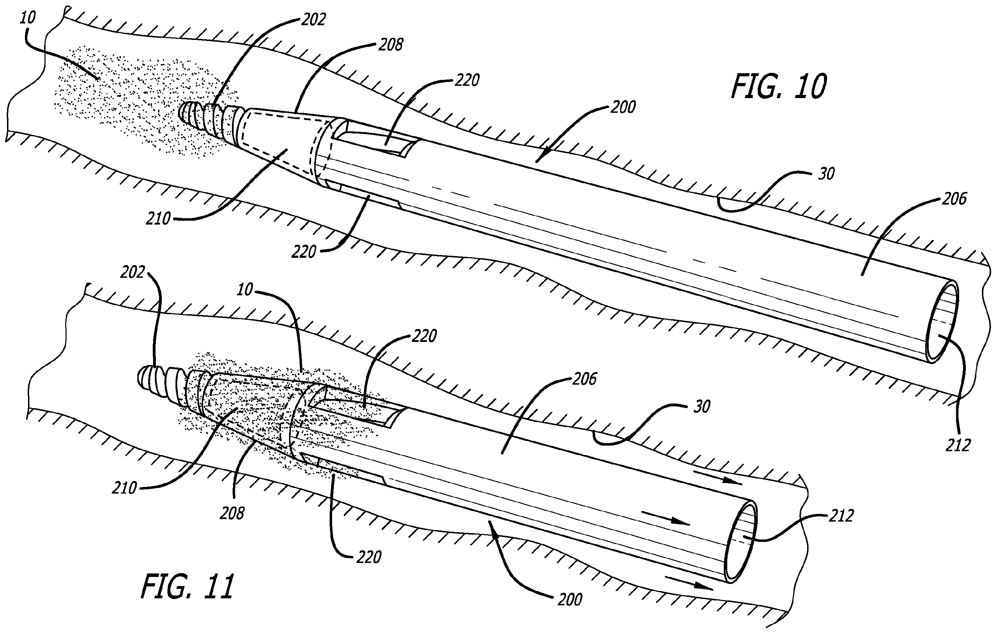

For completeness, a method of using the DAC 200 is described further below and shown in Figs. 10 and 11. However, it should be understood that this method can be performed with any of the embodiments and/or variations described in this specification.

A guidewire and/or delivery sheath (not shown in the drawings with DAC 200) may be initially deployed in the vessel 30 of a patient such that the guidewire or sheath allows the distal tip 202 of the DAC 200 to be positioned near a clot 10. For example, the DAC 200 may be inserted into the targeted blood vessel 30 over the guidewire (e.g., through the aspiration lumen 201 or a separate guidewire lumen of the DAC 200) and advanced towards the clot 10 or the DAC 200 can be advanced within a placed sheath having its distal end placed near the clot 10. The radiopaque marker bands (as shown in previous embodiments) can be used to confirm the proper positioning of the DAC 200 with respect to the clot 10.

The DAC 200 is further distally advanced so that the distal tip 202 is positioned at least partially into the clot 10, as seen in Fig. 10. Optionally, the distal tip 202 may be rotated at least partially to screw into the clot 10, either by rotating the DAC 200 or by rotating the distal tip 202 via a shaft within the DAC 200 (if included).

An aspiration source connected to the proximal end of the aspiration lumen 212 is activated such that the clot 10 is further advanced towards the distal openings 220 and ingested at least partially into the aspiration lumen 212 of the DAC 200 (as shown in Fig. 11).

This may be achieved by static aspiration or through cyclic aspiration. Applying cyclic aspiration at this time may help further fatigue the clot 10, especially if it is sized and structured such that it is resistant to passing into the distal openings 220. Particularly, the water hammer pressure waves from this cyclic aspiration may help create force on the clot 10 such that clot fatigue is induced. Hence, it may be desirable to perform an initial period of cyclic aspiration to create pressure waves to fatigue the clot 10, then apply a period of static aspiration to suction at least part of the clot 10 into the aspiration lumen 212.

Once the clot 10 is at least partially ingested into the aspiration lumen 212 (Fig. 11), an additional period of cyclic aspiration may be performed. The water hammer pressure waves created with this cyclic aspiration may push part or all of the clot 10 distally forward into the interior cavity 210 of the intermediate conical portion 208. This positioning of the clot 10 within the interior cavity 210 helps hold or secure the clot in place, which may be particularly helpful as the DAC 200 is proximally withdrawn from the patient's vessel. In some circumstances, this withdrawal processes can create forces on the clot 10 if a portion of the clot remains outside of the DAC 200 or if distal suction is created by the proximal movement of the DAC 200.

After the DAC 200 is withdrawn, a contrast agent may be injected to determine if the recanalization of the blood vessel was successful.

While specific embodiments and features of these embodiments have been disclosed, it should be understood that any of the features described in this specification can be used in any combination. Hence, while specific embodiments are described as an example, it is intended that the features described herein can be mixed and matched in any manner.

Although the invention has been described in terms of particular embodiments and applications, one of ordinary skill in the art, in light of this teaching, can generate additional embodiments and modifications without departing from the scope of the claimed invention. Accordingly, it is to be understood that the drawings and descriptions herein are offered by way of example to facilitate comprehension of the invention and should not be construed to limit the scope thereof.

## Claims

1. A clot removal device, comprising:
a catheter body (100) having a first lumen positioned inside the catheter body between a proximal portion and a distal portion of the catheter body, and a closed distal tip (102) located at a distal end of the catheter body, the distal tip having a hollow cavity (110) in fluid communication with the first lumen; and,
a distal opening (120) that opens into the first lumen and that is located near a proximal end of the distal tip,
wherein the hollow cavity has a maximum cross-sectional area less than a cross-sectional area of the first lumen.

2. The clot removal catheter of claim 1, further comprising an aspiration source connected to the first lumen and configured to provide cyclic aspiration that alternates between providing higher aspiration force and lower/no aspiration force.

3. The clot removal device of claim 1, wherein the distal opening (120) and the distal tip (102) are positioned on opposite sides of a longitudinal axis of the catheter.

4. The clot removal device of claim 1, wherein the distal opening (120) is positioned at an angle relative to a longitudinal axis of the catheter body (100) in a range of 15 degrees to 315 degrees.

5. The clot removal device of claim 1, wherein the distal opening (120) comprises a shape of a semicircle, a rectangle, a square, or an oval.

6. The clot removal device of claim 1, wherein the distal tip (102) comprises a shape of a cone, a cylinder, a triangle, a rectangle, a helix, or a spiral.

7. The clot removal catheter of claim 1, wherein the distal opening (120) comprises a plurality of distal openings (121).

8. The clot removal catheter of claim 7, wherein the plurality of distal openings (121) are positioned within channels within an outer surface of the catheter (100).

9. The clot removal catheter of claim 1, further comprising a radiopaque marker (124) positioned along an edge of the distal opening (120).

10. The clot removal catheter of claim 1, wherein the distal opening (120) comprises a recessed area with a lower surface angled radially inwardly in a distal direction so as to direct a clot into the hollow cavity (110).

11. The clot removal catheter of claim 8, the plurality of distal openings (121) are positioned either at the same longitudinal positions or at different longitudinal positions from each other.

12. The clot removal catheter of claim 1, wherein the distal opening (120) is between 0 and 90 degrees relative to an axis of the catheter body (100).

13. The clot removal catheter of claim 1, wherein the distal opening (120) proximally increases in height.

14. The clot removal catheter of claim 1, further comprising a recessed area (220A) formed in the catheter body (100), the recessed area being proximally offset from the distal tip (102).

15. The clot removal catheter of claim 14, wherein the recessed area (220A) comprises one or more openings (221) that open into the first lumen (101).

## Patentansprüche

1. Vorrichtung zum Entfernen von Blutgerinnseln, umfassend:
einen Katheterkörper (100) mit einem ersten Lumen, das innerhalb des Katheterkörpers zwischen einem proximalen Abschnitt und einem distalen Abschnitt des Katheterkörpers angeordnet ist, und einer geschlossenen distalen Spitze (102), die sich an einem distalen Ende des Katheterkörpers befindet, wobei die distale Spitze einen Hohlraum (110) aufweist, der in Fluidverbindung mit dem ersten Lumen steht; und
eine distale Öffnung (120), die in das erste Lumen mündet und sich in der Nähe eines proximalen Endes der distalen Spitze befindet,
wobei der Hohlraum eine maximale Querschnittsfläche aufweist, die kleiner als die Querschnittsfläche des ersten Lumens ist.

2. Katheter zum Entfernen von Blutgerinnseln nach Anspruch 1, ferner umfassend eine Aspirationsquelle, die mit dem ersten Lumen verbunden ist und so konfiguriert ist, dass sie eine zyklische Aspiration bereitstellt, die zwischen einer höheren Aspirationskraft und einer geringeren/keiner Aspirationskraft wechselt.

3. Vorrichtung zum Entfernen von Blutgerinnseln nach Anspruch 1, wobei die distale Öffnung (120) und die distale Spitze (102) auf gegenüberliegenden Seiten einer Längsachse des Katheters positioniert sind.

4. Vorrichtung zum Entfernen von Blutgerinnseln nach Anspruch 1, wobei die distale Öffnung (120) in einem Winkel relativ zu einer Längsachse des Katheterkörpers (100) in einem Bereich von 15 Grad bis 315 Grad positioniert ist.

5. Vorrichtung zum Entfernen von Blutgerinnseln nach Anspruch 1, wobei die distale Öffnung (120) die Form eines Halbkreises, eines Rechtecks, eines Quadrats oder eines Ovals aufweist.

6. Vorrichtung zum Entfernen von Blutgerinnseln nach Anspruch 1, wobei die distale Spitze (102) die Form eines Kegels, eines Zylinders, eines Dreiecks, eines Rechtecks, einer Helix oder einer Spirale aufweist.

7. Katheter zum Entfernen von Blutgerinnseln nach Anspruch 1, wobei die distale Öffnung (120) eine Vielzahl von distalen Öffnungen (121) umfasst.

8. Katheter zum Entfernen von Blutgerinnseln nach Anspruch 7, wobei die Vielzahl von distalen Öffnungen (121) innerhalb von Kanälen innerhalb einer Außenoberfläche des Katheters (100) positioniert sind.

9. Katheter zum Entfernen von Blutgerinnseln nach Anspruch 1, ferner umfassend eine röntgendichte Markierung (124), die entlang einer Kante der distalen Öffnung (120) positioniert ist.

10. Katheter zum Entfernen von Blutgerinnseln nach Anspruch 1, wobei die distale Öffnung (120) eine vertiefte Fläche mit einer unteren Oberfläche umfasst, die radial nach innen in distaler Richtung abgewinkelt ist, um ein Blutgerinnsel in den Hohlraum (110) zu leiten.

11. Katheter zum Entfernen von Blutgerinnseln nach Anspruch 8, wobei die Vielzahl von distalen Öffnungen (121) entweder an denselben Längspositionen oder an voneinander unterschiedlichen Längspositionen voneinander positioniert sind.

12. Katheter zum Entfernen von Blutgerinnseln nach Anspruch 1, wobei die distale Öffnung (120) relativ zu einer Achse des Katheterkörpers (100) zwischen 0 und 90 Grad liegt.

13. Katheter zum Entfernen von Blutgerinnseln nach Anspruch 1, wobei die distale Öffnung (120) proximal in der Höhe zunimmt.

14. Katheter zum Entfernen von Blutgerinnseln nach Anspruch 1, ferner umfassend eine in den Katheterkörper (100) eingeformte vertiefte Fläche (220A), wobei die vertiefte Fläche proximal von der distalen Spitze (102) versetzt ist.

15. Katheter zum Entfernen von Blutgerinnseln nach Anspruch 14, wobei die vertiefte Fläche (220A) eine oder mehrere Öffnungen (221) umfasst, die in das erste Lumen (101) münden.

## Revendications

1. Dispositif d'extraction de caillot comprenant :
un corps de cathéter (100) présentant une première lumière située à l'intérieur du corps de cathéter, entre une partie proximale et une partie distale du corps de cathéter, et une pointe distale fermée (102) située à l'extrémité distale du corps de cathéter, la pointe distale comportant une cavité creuse (110) en communication fluidique avec la première lumière ; et
une ouverture distale (120) qui débouche dans la première lumière et qui est située près d'une extrémité proximale de la pointe distale,
dans lequel la cavité creuse présente une surface de section transversale maximale inférieure à une surface de section transversale de la première lumière.

2. Cathéter d'extraction de caillot selon la revendication 1, comprenant en outre une source d'aspiration reliée à la première lumière et conçue pour fournir une aspiration cyclique alternant entre la fourniture d'une force d'aspiration plus élevée et d'une force d'aspiration plus faible, voire nulle.

3. Dispositif d'extraction de caillot selon la revendication 1, dans lequel l'ouverture distale (120) et la pointe distale (102) sont positionnées de part et d'autre d'un axe longitudinal du cathéter.

4. Dispositif d'extraction de caillot selon la revendication 1, dans lequel l'ouverture distale (120) est positionnée selon un angle par rapport à un axe longitudinal du corps du cathéter (100) dans une plage de 15 degrés à 315 degrés.

5. Dispositif d'extraction de caillot selon la revendication 1, dans lequel l'ouverture distale (120) a la forme d'un demi-cercle, d'un rectangle, d'un carré ou d'un ovale.

6. Dispositif d'extraction de caillot selon la revendication 1, dans lequel la pointe distale (102) a la forme d'un cône, d'un cylindre, d'un triangle, d'un rectangle, d'une hélice ou d'une spirale.

7. Cathéter d'extraction de caillot selon la revendication 1, dans lequel l'ouverture distale (120) comprend une pluralité d'ouvertures distales (121).

8. Cathéter d'extraction de caillot selon la revendication 7, dans lequel la pluralité d'ouvertures distales (121) sont positionnées à l'intérieur de canaux situés dans la surface externe du cathéter (100).

9. Cathéter d'extraction de caillot selon la revendication 1, comprenant en outre un marqueur radio-opaque (124) positionné le long d'un bord de l'ouverture distale (120).

10. Cathéter d'extraction de caillot selon la revendication 1, dans lequel l'ouverture distale (120) comprend une zone en retrait dont la surface inférieure est inclinée radialement vers l'intérieur, dans une direction distale, de sorte à diriger un caillot dans la cavité creuse (110).

11. Cathéter d'extraction de caillot selon la revendication 8, dans lequel la pluralité d'ouvertures distales (121) sont positionnées soit à la même position longitudinale, soit à des positions longitudinales différentes les unes des autres.

12. Cathéter d'extraction de caillot selon la revendication 1, dans lequel l'ouverture distale (120) forme un angle compris entre 0 et 90 degrés par rapport à un axe du corps du cathéter (100).

13. Cathéter d'extraction de caillot selon la revendication 1, dans lequel l'ouverture distale (120) présente une hauteur proximalement croissante.

14. Cathéter d'extraction de caillot selon la revendication 1, comprenant en outre une zone en retrait (220A) formée dans le corps de cathéter (100), la zone en retrait étant décalée de manière proximale par rapport à la pointe distale (102).

15. Cathéter d'extraction de caillot selon la revendication 14, dans lequel la zone en retrait (220A) comprend une ou plusieurs ouvertures (221) qui débouchent dans la première lumière (101).
